**Europäisches Patentamt**

**European Patent Office**

**Office européen des brevets**

(11) Numéro de publication : **0 447 298 B1**

(12) **FASCICULE DE BREVET EUROPEEN**

(45) Date de publication du fascicule du brevet :
08.06.94 Bulletin 94/23

(51) Int. Cl.$^5$ : **A61K 9/52,** A61K 9/32

(21) Numéro de dépôt : **91400613.5**

(22) Date de dépôt : **06.03.91**

(54) **Procédé d'enrobage par un polymère PH sensible de principes actifs.**

(30) Priorité : **08.03.90 FR 9002973**

(43) Date de publication de la demande :
**18.09.91 Bulletin 91/38**

(45) Mention de la délivrance du brevet :
**08.06.94 Bulletin 94/23**

(84) Etats contractants désignés :
**AT BE CH DE DK ES FR GB GR IT LI LU NL SE**

(56) Documents cités :
**EP-A- 0 188 953
EP-A- 0 260 186
EP-A- 0 336 713
WO-A-80/00659**

(73) Titulaire : **RHONE-POULENC NUTRITION
ANIMALE
Rue Marcel Lingot
F-03600 Commentry (FR)**

(72) Inventeur : **Ardaillon, Pierre
10 Impasse Beau-Vallon
F-69800 Saint-Priest (FR)**
Inventeur : **Prud'Homme, Christian
19 rue Commandant Faurax
F-69006 Lyon (FR)**

(74) Mandataire : **Le Pennec, Magali et al
RHONE-POULENC RORER SA,
Direction des Brevets,
20 Avenue Raymond Aron
F-92160 Antony (FR)**

## Description

La présente invention concerne un procédé d'enrobage de principes actifs, par une formulation contenant un ou plusieurs polymères PH sensibles. Elle concerne plus particulièrement un procédé d'enrobage, par une émulsion ou une suspension aqueuse d'un ou plusieurs polymères PH sensibles, de principes actifs médicamenteux et/ou alimentaires destinés à l'alimentation des ruminants.

Il est connu depuis longtemps d'enrober des principes actifs par des polymères PH sensibles, éventuellement mélangés avec une substance hydrophobe, telle que l'acide stéarique, et/ou un polymère non hydrosoluble tel que l'éthylcellulose. Ces enrobages sont particulièrement décrits en ce qui concerne l'alimentation animale dans les demandes de brevets publiées sous les numéros US 4 832 967, EP 260 186 ou EP 188 953.

Selon ces demandes on enrobe les particules sphériques de méthionine et/ou de lysine par la technique du lit fluidisé à l'aide d'une solution de polymère PH sensible dissoute dans un ou plusieurs solvants organiques tels que les solvants halogénés, les alcools ou les éthers. Cette technique permet une pulvérisation facile du ou des polymères PH sensibles mais présente l'inconvénient d'introduire une quantité importante de solvants, environ 20 à 100 g de solvants pour 1 g de polymère PH sensible. Il est regrettable de devoir introduire sur des produits destinés à l'alimentation animale des solvants dont l'écotoxicité n'est pas reconnue.

Le brevet EP-A-0260186 décrit un procédé d'enrobage par un ou plusieurs polymères pH sensibles de granulés de principes actifs médicamenteux et/ou alimentaires par une solution organique ou une émulsion aqueuse du ou des polymères pH sensibles. Les seuls émulsions aqueuses décrites contiennent (exemples 6 et 7) respectivement 6,7 et 12 % de solvant. Ces émulsions correspondent surtout pour celle de l'exemple 8 à l'émulsion utilisée dans l'exemple 3 de la présente demande (même concentration en solvant et même nature du solvant). Cette émulsion n'est utilisable qu'immédiatement, après stockage de l'émulsion, la protection des principes actifs enrobés par cette émulsion est mauvaise (75 % de libération du principe actif en 6 heures à pH6).

La présente invention a permis d'enrober, à l'aide de polymères PH sensibles, des principes actifs tels que les acides aminés ou les vitamines en évitant au maximum l'usage de solvants organiques non compatibles avec les milieux biologiques.

En effet la présente invention concerne un procédé d'enrobage, par un ou plusieurs polymères PH sensibles, de principes actifs médicamenteux et/ou alimentaires destinés à l'alimentation des ruminants, par pulvérisation sur ceux-ci d'une émulsion ou d'une dispersion aqueuse du ou des polymères PH sensibles contenant moins de 0,5 % en poids de solvant organique et ceci malgré l'hydrosolubilité importante de certains principes actifs à enrober comme notamment la lysine ou ses dérivés. Les principes actifs médicamenteux et/ou alimentaires à enrober se présentent de préférence sous forme de granulés ayant un diamètre compris entre 0,3 et 3 mm.

Le dépôt de l'enrobant à partir de l'émulsion aqueuse permet une économie importante dans la mise en oeuvre du procédé, car elle évite l'utilisation d'installations de récupération de solvants, coûteuses du point de vue sécurité et investissement. Elle permet aussi d'augmenter les productivités par rapport aux procédés utilisés dans l'art antérieur.

Les polymères PH sensibles, utilisés dans le procédé selon l'invention, sont choisis notamment parmi:

- les polyvinyl acétals d'esters acétylacétiques substitués par des groupes azotés dialkylés tels que le groupe diéthylamino,
- les copolymères des dialkylaminoalkylacrylates et méthacrylates et d'un ester ou d'un acide acrylique ou méthacrylique,
- les copolymères du styrène ou de l'acrylonitrile avec les isomères ou les dérivés de la vinylpyridine.

On préfère parmi l'ensemble des polymères cités utiliser les copolymères du styrène avec la vinyl-2 pyridine.

Les principes actifs médicamenteux sont choisis notamment parmi les vitamines, les antibiotiques, les composés antiparasitaires, les hormones. Les principes actifs alimentaires sont choisis notamment parmi les acides aminés essentiels supposés limitant tels que la méthionine et/ou la lysine et/ou le tryptophane.

On peut adjoindre à ces principes actifs des charges, éventuellement lamellaires, qui facilitent le délitement dans le tractus digestif. Ces charges sont notamment choisies parmi les charges PH sensibles ou non telles que par exemple: le talc et/ou la silice et/ou les carbonates et/ou les polyphosphates complexes tels que à base de $Na_2O$, $CaO$, $P_2O_5$ et $Al_2O_3$. On peut également adjoindre à ces principes actifs des agents liants choisis parmi les acides ou les esters gras, la cellulose (telle que celle commerciallisée, par exemple, sous la dénomination Avicel) ou ses dérivés, notamment l'éthylcellulose, la carboxyméthylcellulose, ainsi que les copolymères basiques. L'ensemble du ou des principes actifs et des additifs forment le coeur du granulé qui est ensuite enrobé par le ou les polymères PH sensible pulvérisé sous forme d'une émulsion aqueuse.

Ces principes actifs médicamenteux ou alimentaires avec éventuellement un additif et enrobés de polymère PH sensible sont particulièrement intéressants pour l'alimentation des ruminants car ils sont peu ou pas dégradés lors du transit dans le rumen et sont libérés dans la caillette et/ou l'intestin des ruminants.

L'enrobage est réalisé par pulvérisation d'une émulsion ou d'une dispersion aqueuse contenant le ou les polymères PH sensibles. Cette émulsion ou cette dispersion est réalisée par mélange d'une solution organique contenant le ou les polymères PH sensibles et d'une solution aqueuse contenant un émulsifiant.

L'émulsion d'enrobage peut aussi contenir des additifs tels que ceux mentionnés précédemment, ainsi que des agents antistatiques, des agents plastifiants, des colorants ou des agents d'apétence. On préfère utiliser une dispersion aqueuse contenant le ou les polymères PH sensibles, une substance hydrophobe et contenant éventuellement un polymère non hydrosoluble.

La substance hydrophobe est choisie de préférence parmi les acides gras contenant 12 à 22 atomes de carbone tels que par exemple l'acide stéarique ou l'acide béhénique. L'émulsifiant peut être choisi parmi les esters d'acides gras ou les sels d'acides gras. L'émulsifiant peut dans ce dernier cas être créé "in situ" par salification de l'acide gras au moyen d'une base choisie parmi les hydroxydes alcalins et d'ammonium.

Le polymère non hydrosoluble est avantageusement choisi parmi les ethers et les esters de cellulose non hydrosolubles tels que l'éthylcellulose, l'acétate de cellulose, le propionate de cellulose, l'acétobutyrate de cellulose.

Le solvant organique du ou des polymères PH sensibles est choisi parmi les alcools linéaires ou ramifiés contenant au moins quatre atomes de carbone, les esters tels que les acétates d'alkyle, les éthers tels que par exemple l'éther isopropylique, les cétones telles que la méthylisobutylcétone et les solvants halogénés tels que le tetrachloroéthane, le trichloroéthane, le dichloroéthane, le chloroforme et le dichlorométhane.

L'émulsion est préparée par mélange d'une phase aqueuse majoritaire et d'une phase organique. D'un point de vue mise en oeuvre, on préfère que la phase aqueuse représente 97 à 60 % en volume et la phase organique représente 3 à 40 % en volume.

La phase aqueuse contient l'émulsifiant et/ou un de ses précurseurs permettant la formation "in situ" de l'émulsifiant. Ainsi elle contient notamment 0 à 10 % en poids d'un émulsifiant choisi, comme mentionné précédemment, parmi les esters ou les sels d'acides gras et/ou elle contient 0 à 1 % en poids d'un hydroxyde alcalin ou d'ammonium. Il est évident que pour que l'émulsion se forme, au moins l'un des deux groupes de constituants, soit l'émulsifiant, soit l'hydroxyde alcalin ou d'ammonium et l'acide gras doit être présent. La quantité minimale d'émulsifiant additionné ou formé "in situ" calculée sur la base de l'hydroxyde de sodium est de 0,2 % en poids.

La phase organique est obtenue par dispersion ou mise en solution d'un mélange solide ayant de préférence la constitution pondérale suivante:

- polymère(s) PH sensible(s) 10 à 70 %
- une ou plusieurs substances hydrophobes 30 à 90 %
- un ou plusieurs polymères non hydrosolubles 0 à 20 % dans le solvant du polymère PH sensible.

Le pourcentage pondéral des différentes phases mises en oeuvre est compris notamment dans les limites suivantes:

- phase aqueuse 50 à 97 %
- phase solide 2 à 30 %
- solvant organique 1 à 20 %.

Encore plus préférentiellement les pourcentages pondéraux des différentes phases sont compris dans les limites suivantes:

- phase aqueuse 72 à 87 %
- phase solide 10 à 20 %
- solvant organique 3 à 8 %.

La phase organique, obtenue après dispersion ou mise en solution de la phase solide dans le solvant organique est mélangée avec la phase aqueuse préparée précédemment. Il est évident que lorsque la phase organique ne contient pas d'acide gras, la phase aqueuse contiendra obligatoirement un émulsifiant, par contre lorsque la phase organique contient un acide gras, la phase aqueuse peut contenir un hydroxyde alcalin ou d'ammonium et/ou un émulsifiant.

L'émulsion est réalisée indifféremment par introduction de la phase organique dans la phase aqueuse ou inversement.

Après obtention de l'émulsion celle ci est soumise à une évaporation ou à une distillation de façon à éliminer presque la totalité du solvant organique. Cette élimination est effectuée à une température comprise entre 70 et 100°C,lorsqu'on opère à pression atmosphérique. On peut opérer à des températures inférieures si la pression est inférieure à la pression atmosphérique. L'homme de l'art adaptera les conditions opérationnelles, température, pression et durée de l'opération, à l'économie du procédé. En fin de distillation on obtient notamment une émulsion contenant moins de 30 % d'extrait sec, elle contient moins de 0,5 % de solvant organique et de préférence moins de 0,1 %. Elle est ensuite pulvérisée sur un lit de granulés à enrober par la technique du lit fluidisé de type par exemple WURSTER telle que décrite dans les brevets US 2 799 241 et EP 188 953.

Les granulés obtenus après enrobage sont utilisés pour l'alimentation ou le traitement médicamenteux des ruminants.

Leur préparation sera plus complètement décrite à l'aide des exemples suivants.

EXEMPLE 1

Dans un récipient chauffé et équipé d'un système d'agitation magnétique, on mélange:
- 80 g d'acétate de butyle
- 88 g d'acide stéarique PRIFRAC 2981® ( commercialisé par UNICHEMA ).

Après avoir observé la dissolution complète de l'acide stéarique, on ajoute au mélange:
- 22 g d'un copolymère de vinyl-2-pyridine ( 57 % en poids ) et de styrène ( 43 % en poids ).

Ce copolymère est caractérisé par sa viscosité inhérente: 1,36 dl/g (mesurée à 25°C, en solution dans le diméthylformamide, à la concentration de 0,5 g de copolymère pour 100 ml).

Après quelques minutes de chauffage vers 90-100°C, on obtient une solution homogène.

Dans un autre récipient d'une capacité de 2 litres, on charge 495 g d'eau déminéralisée, puis on ajoute 4,2 ml d'une solution de soude aqueuse à 10 %.

Le contenu du récipient est ensuite chauffé vers 85°C et brassé par un appareil de type POLYTRON tournant à 11000 tours par minute. On ajoute alors, en sept minutes la solution organique précédente préalablement chargée dans une ampoule de coulée maintenue à 95°C.

Après la fin de la coulée, l'émulsion obtenue est diluée avec 495 g d'eau déminéralisée et brassée avec le Polytron pendant encore quatre minutes environ.

La quasitotalité de l'acétate de butyle présent dans l'émulsion, est ensuite éliminée de la manière suivante: ce mélange est maintenu sous agitation magnétique et porté à l'ébullition jusqu'à ce que l'on enregistre une perte de poids due à l'évaporation, égale à 34 % de poids de l'émulsion initiale. En fin d'opération, la teneur pondérale du mélange en extrait sec est ramenée à 10 % par dilution avec de l'eau déminéralisée.

Par analyse chromatographie en phase vapeur, on établit alors que la concentration pondérale résiduelle de l'acétate de butyle dans l'émulsion est inférieure à 0,15 %. L'émulsion est conservée pendant 18 heures, à 70-75°C, sous agitation magnétique, avant d'être utilisée.

Dans un appareil de spray-coating UNIGLATT® équipé d'un système WURSTER, on charge 500 g de granulés sphériques de 2 mm de diamètre moyen et titrant 57 % de chlorhydrate de lysine et 16 % de méthionine.

L'émulsion maintenue à 87°C et doucement agitée, est pulvérisée dans le lit fluidisé formé par les granulés, les conditions de pulvérisation étant les suivantes:
- débit de l'air de fluidisation: 130 m³/h
- température de l'air de fluidisation (sortie):37°C
- pression de l'air de pulvérisation: 1,5bars
- température de l'air de pulvérisation: 83°C
- débit de l'émulsion: 12ml/mn
- durée de pulvérisation: 51mn.

Le degré de protection apporté par la pellicule d'enrobage ainsi déposée est évalué selon un test in vitro consistant à agiter une quantité déterminée de granulés enrobés, dans une solution aqueuse tamponnée à PH6 et maintenue à 40°C. On effectue un dosage de la quantité de chlorhydrate de lysine libérée dans le milieu après 6 heures et 24 heures. Les résultats sont indiqués dans le tableau I.

EXEMPLE 2

On reproduit l'exemple 1, avec les modifications suivantes:
- on élimine l'acétate de butyle par évaporation, jusqu'à constater une perte de poids de l'émulsion égale à 47 % du poids initial.
- Après l'évaporation de l'acétate de butyle, l'émulsion n'est pas rediluée, afin de conserver un taux d'extrait sec de 20 %, en poids pour l'enrobage.
- La concentration pondérale en acétate de butyle résiduel, après évaporation, est inférieure à 0,2 % (par analyse par chromatographie phase vapeur ).
- L'émulsion est utilisée immédiatement après sa préparation pour réaliser un enrobage d'acides aminés dans des conditions identiques à celles qui sont décrites dans l'exemple 1.
- La durée de pulvérisation n'est plus que de 34 minutes, pour un débit d'émulsion de 9,3 ml/mn.

EP 0 447 298 B1

On obtient des granulés présentant une bonne résistance au milieu aqueux à PH6 et à 40°C ( tableau 1 ).

EXEMPLE 3 (comparatif)

A titre d'exemple comparatif, on reproduit strictement l'exemple 1 en supprimant la phase d'évaporation et de stockage. L'émulsion obtenue est utilisée immédiatement après sa préparation pour enrober les granulés d'acides aminés décrits précédemment.

EXEMPLE 4 (comparatif)

Afin de montrer que l'élimination du solvant organique conduit à des émulsions plus stables, on reproduit strictement l'exemple 3 et l'on stocke l'émulsion pendant 19 heures à 72°C avant de l'utiliser pour réaliser un enrobage dans les mêmes conditions que dans l'exemple 1.

La mauvaise qualité du produit ( tableau 1 ) indique une moindre stabilité de l'émulsion par rapport à l'exemple 1.

EXEMPLE 5

Dans un récipient chauffé et équipé d'un système d'agitation magnétique, on mélange :
- 80 g d'acétate de butyle,
- 88 g d'acide stéarique PRIFRAC$^R$2981.

Après la dissolution complète de l'acide stéarique, on ajoute au mélange :
- 22 g d'un copolymère de vinyl-2-pyridine (70 % en poids, et de styrène (30 % en poids).

Ce copolymère est caractérisé par sa viscosité inhérente $[\mu]$inhr = 1,20 dl/g (à 25°C, en solution dans le diméthylformamide à la concentration de 0,5 g pour 100 ml).

Après quelques minutes de chauffage à 90-100°C, on obtient une solution homogène.

Dans un autre récipient d'une capacité de 2 litres, on charge 495 g d'eau déminéralisée additionnée de 4,2 ml d'une solution aqueuse de soude à 10 %.

Le contenu de ce récipient est ensuite chauffé à 85°C et brassé par la turbine d'un appareil de type POLYTRON tournant à 11000 tours par minute.

On ajoute alors, en sept minutes, la solution organique précédente préalablement chargée dans une ampoule de coulée maintenue à 95°C.

Après la coulée, l'émulsion obtenue est diluée avec 104 g d'eau déminéralisée, et brassée pendant encore 4 minutes avec la turbine POLYTRON.

La quasitotalité de l'acétate de butyle présent dans l'émulsion est ensuite éliminée de la manière suivante :

Le récipient dans lequel l'émulsion est maintenue sous agitation magnétique, est équipé d'une colonne à distiller, d'un réfrigérant descendant et d'un récepteur.

Après distillation à la pression atmosphérique, on recueille dans le récepteur un mélange formé des deux phases liquides immiscibles et limpides, représentant au total 18 % en poids de l'émulsion initiale.

La teneur pondérale en extrait sec de l'émulsion ainsi obtenue est de 21 %.

Sa concentration résiduelle en acétate de butyle, déterminée par chromatographie en phase vapeur, est de 0,05 %.

L'émulsion est conservée pendant 19 heures, à 70-75°C, sous agitation magnétique douce avant d'être pulvérisée dans un appareil de spray-coating UNIGLATT$^R$ équipé d'un système WURSTER et chargé avec 500 g de granulés d'acides aminés identiques à ceux de l'exemple 1.

Durant la pulvérisation, on maintient l'agitation de l'émulsion alors chauffée à 87°C.

Les conditions de pulvérisation étant les suivantes :
- débit de l'air de fluidisation : 130 m³/h
- température de l'air de fluidisation (sortie) : 37°C
- pression de l'air de pulvérisation : 1,5 bar
- température de l'air de pulvérisation : 87°C
- débit d'émulsion : 9 ml/minute
- durée de pulvérisation : 39 minutes

Les granulés obtenus sont caractérisés de la même manière que dans les exemples précédents.

Ces résultats sont indiqués dans le Tableau I.

5

TABLEAU I

| Echantillon | Emulsion | | Taux d'enrobant % | Test in vitro:PH6/40°C | | Test in vitro:PH2/40°C | |
|---|---|---|---|---|---|---|---|
| | % en poids d'acétate de butyle | Durée de stockage (H) avant utilisation | | % de lysine libérée après | | % de lysine libérée après | |
| | | | | 6 H | 24 H | 15 mn | 30 mn |
| Exemple 1 | 0,13 | 18 | 10,4 | 1,9 | 3,7 | 87 | 100 |
| Exemple 2 | 0,2 | 0 | 8,7 | 1,4 | 2,7 | 90 | 100 |
| Exemple 3 | 3,9 | 0 | 9,5 | 0,5 | 1,5 | 83 | 100 |
| Exemple 4 | 2,5 | 19 | 10,4 | 75 | 92 | 92 | 100 |
| Exemple 5 | 0,05 | 19 | 11,6 | 0,5 | 2,3 | 90 | 100 |

EP 0 447 298 B1

## Revendications

1. Procédé d'enrobage par un ou plusieurs polymères PH sensibles de principes actifs médicamenteux et/ou alimentaires destinés à l'alimentation des ruminants caractérisé en ce que l'on pulvérise sur ce ou ces principes actifs une émulsion ou une dispersion aqueuse du ou des polymères PH sensibles contenant moins de 0,5 % en poids de solvant organique.

2. Procédé selon la revendication 1 caractérisé en ce que l'émulsion à pulvériser contient moins de 0,1 % en poids de solvant organique.

3. Procédé selon la revendication 1 caractérisé en ce que l'émulsion ou la dispersion aqueuse du ou des polymères PH sensibles est obtenue par mélange d'une phase organique contenant le ou les polymères PH sensibles et une substance hydrophobe et éventuellement un polymère non hydrosoluble et d'une solution aqueuse contenant un émulsifiant ou un de ses précurseurs permettant la formation "in situ" de l'émulsifiant, suivi d'une évaporation ou d'une distillation du solvant organique.

4. Procédé selon la revendication 1 caractérisé en ce que le principe actif est un acide aminé choisi parmi la méthionine et/ou la lysine ou un de ses dérivés.

5. Procédé selon la revendication 3 caractérisé en ce que la substance hydrophobe est choisie parmi les acides gras contenant 12 à 22 atomes de carbone et de préférence est l'acide stéarique.

6. Procédé selon la revendication 3 caractérisé en ce que le polymère non hydrosoluble est choisi parmi les éthers et les esters de cellulose non hydrosolubles.

7. Procédé selon la revendication 3 caractérisé en ce que l'émulsifiant est choisi parmi les sels et les esters d'acides gras.

8. Procédé selon la revendication 3 caractérisé en ce que le précurseur de l'émulsifiant permettant la formation de l'émulsifiant "in situ" est choisi parmi les hydroxydes alcalins ou d'ammonium.

9. Procédé selon la revendication 1 caractérisé en ce qu'on ajoute au principe actif une charge choisie parmi les charges telles que : le talc et/ou la silice et/ou les carbonates et/ou les polyphosphates complexes tels que à base de $Na_2O$, $CaO$, $P_2O_5$ et $Al_2O_3$.

10. Procédé selon l'une quelconque des revendications précédentes caractérisé en ce que l'on pulvérise sur des granulés de méthionine et/ou de lysine une émulsion constituée d'un mélange de polymère PH sensible, d'éthylcellulose, d'acide stéarique, de stéarate de sodium et d'eau.

11. Procédé selon les revendications 3 et 10 caractérisé en ce que l'émulsion contient en plus un ou plusieurs additifs choisis parmi les charges telles que définies à la revendication 9, des agents antistatiques, des agents plastifiants, des colorants et des agents d'apétence.

12. Procédé selon l'une quelconque des revendications précédentes caractérisé en ce que l'émulsion est préparée par mélange de 97 à 60 % en volume d'une phase aqueuse et de 3 à 40 % en volume d'une phase organique.

13. Procédé selon la revendication 12 caractérisé en ce que la phase aqueuse contient 0 à 10 % en poids d'un émulsifiant selon la revendication 7 et/ou elle contient 0 à 1 % en poids d'un hydroxyde alcalin ou d'ammonium, la somme des deux représentant au moins 0,2 % en poids d'équivalent hydroxyde de sodium.

14. Procédé selon la revendication 3 caractérisé en ce que la phase organique est obtenue par dispersion ou mise en solution d'un mélange solide ayant la constitution pondérale suivante:
    - polymère PH sensible 10 à 70 %
    - une ou plusieurs substances hydrophobes 30 à 90 %
    - un ou plusieurs polymères non hydrosolubles 0 à 20 % dans un solvant du ou des polymères PH sensibles.

15. Procédé selon les revendications 3 et 14 caractérisé en ce que le pourcentage pondéral des différents

EP 0 447 298 B1

constituants est compris dans les limites suivantes:
- phase aqueuse 50 à 97 %
- mélange solide 2 à 30 %
- solvant organique 1 à 20 %.

**16.** Procédé selon la revendication 15 caractérisé en ce que le pourcentage pondéral des différents constituants est compris dans les limites suivantes:
- phase aqueuse 72 à 87 %
- mélange solide 10 à 20 %
- solvant organique 3 à 8 %.


**Claims**

**1.** Method for coating medicament and/or foodstuff active principles intended for feeding ruminants with one or more pH-sensitive polymers, characterized in that an aqueous emulsion or dispersion of the pH-sensitive polymer or polymers containing less than 0.5% by weight of organic solvent is sprayed onto said active principle or principles.

**2.** Method according to Claim 1, characterized in that the emulsion to be sprayed contains less than 0.1 % by weight of organic solvent.

**3.** Method according to Claim 1, characterized in that the aqueous emulsion or dispersion of the pH-sensitive polymer or polymers is obtained by mixing an organic phase, containing the pH-sensitive polymer or polymers and a hydrophobic substance and optionally a water-insoluble polymer, and an aqueous solution, containing an emulsifier or one of its precursors permitting "in situ" formation of the emulsifier, followed by an evaporation or a distillation of the organic solvent.

**4.** Method according to Claim 1, characterized in that the active principle is an amino acid chosen from methionine and/or lysine or one of its derivatives.

**5.** Method according to Claim 3, characterized in that the hydrophobic substance is chosen from fatty acids containing 12 to 22 carbon atoms and is preferably stearic acid.

**6.** Method according to Claim 3, characterized in that the water-insoluble polymer is chosen from water-insoluble cellulose ethers and esters.

**7.** Method according to Claim 3, characterized in that the emulsifier is chosen from fatty acid salts and esters.

**8.** Method according to Claim 3, characterized in that the emulsifier precursor permitting "in situ" formation of the emulsifier is chosen from alkali metal hydroxides or ammonium hydroxide.

**9.** Method according to Claim 1, characterized in that a filler chosen from fillers such as: talc, and/or silica and/or carbonates and/or complex polyphosphates, such as those based on $Na_2O$, $CaO$, $P_2O_5$ and $Al_2O_3$, is added to the active principle.

**10.** Method according to any one of the preceding Claims, characterized in that an emulsion consisting of a mixture of pH-sensitive polymer, ethylcellulose, stearic acid, sodium stearate and water is sprayed onto granules of methionine and/or lysine.

**11.** Method according to Claims 3 and 10, characterized in that the emulsion also contains one or more additives chosen from fillers such as defined in Claim 9, antistatic agents, plasticizers, colorants and appetite stimulants.

**12.** Method according to any one of the preceding Claims, characterized in that the emulsion is prepared by mixing 97 to 60 % by volume of an aqueous phase and 3 to 40 % by volume of an organic phase.

**13.** Method according to Claim 12, characterized in that the aqueous phase contains 0 to 10 % by weight of an emulsifier according to Claim 7 and or it contains 0 to 1 % by weight of an alkali metal hydroxide or

EP 0 447 298 B1

ammonium hydroxide, the sum of the two representing at least 0.2 % by weight of sodium hydroxide equivalent.

14. Method according to Claim 3, characterized in that the organic phase is obtained by dispersing or dissolving a solid mixture having the following composition by weight:
    - 10 to 70 % of pH-sensitive polymer,
    - 30 to 90 % of one or more hydrophobic substances, and
    - 0 to 20 % of one or more water-insoluble polymers, in a solvent for the pH-sensitive polymer or polymers.

15. Method according to Claims 3 and 14, characterized in that the percentage by weight of the various constituents is within the following limits:
    - aqueous phase 50 to 97 %,
    - solid mixture 2 to 30 %, and
    - organic solvent 1 to 20 %.

16. Method according to Claim 15, characterized in that the percentage by weight of the various constituents is within the following limits:
    - aqueous phase 72 to 87 %,
    - solid mixture 10 to 20 %, and
    - organic solvent 3 to 8 %.

**Patentansprüche**

1. Verfahren zur Umhüllung von medikamentösen Wirkstoffen und/oder Nahrungsstoffen. die für die Ernährung von Wiederkäuern vorgesehen sind, durch ein oder mehrere pH-sensible Polymere, dadurch gekennzeichnet. daß man auf dem oder den Wirkstoff(en) eine wäßrige Emulsion oder Dispersion des oder der pH-sensiblen Polymere versprüht, die weniger als 0.5 Gew.-% organisches Lösungsmittel enthalten.

2. Verfahren nach Anspruch 1, dadurch gekennzeichnet, daß die zu versprühende Emulsion weniger als 0,1 Gew.-% organisches Lösungsmittel enthält.

3. Verfahren nach Anspruch 1, dadurch gekennzeichnet, daß die wäßrige Emulsion oder Dispersion des oder der pH-sensiblen Polymere durch Mischen einer organischen Phase, die das oder die pH-sensiblen Polymere und eine hydrophobe Substanz und gegebenenfalls ein nicht wasserlösliches Polymer enthält, mit einer wäßrigen Lösung, die einen Emulgator oder einen ihrer Vorläufer enthält, der die Bildung des Emulgators "in situ" ermöglicht, und nachfolgende Verdampfung oder Destillation des organischen Lösungsmittels erhalten wird.

4. Verfahren nach Anspruch 1, dadurch gekennzeichnet, daß der Wirkstoff eine Aminosäure ist, ausgewählt unter Methionin und/oder Lysin und ihren Derivaten.

5. Verfahren nach Anspruch 3, dadurch gekennzeichnet, daß die hydrophobe Substanz ausgewählt wird unter den Fettsäuren mit 12 bis 22 Kohlenstoffatomen und vorzugsweise Stearinsäure ist.

6. Verfahren nach Anspruch 3, dadurch gekennzeichnet, daß das nicht wasserlösliche Polymer ausgewählt wird unter den nicht wasserlöslichen Ethern und Estern von Cellulose.

7. Verfahren nach Anspruch 3, dadurch gekennzeichnet. daß der Emulgator ausgewählt wird unter den Salzen und Estern von Fettsäuren.

8. Verfahren nach Anspruch 3, dadurch gekennzeichnet, daß der Vorläufer des Emulgators, der die Bildung des Emulgators "in situ" ermöglicht, ausgewählt wird unter den Alkalihydroxiden oder Ammoniumhydroxid.

9. Verfahren nach Anspruch 1, dadurch gekennzeichnet, daß man dem Wirkstoff einen Füllstoff zugibt, ausgewählt unter Füllstoffen wie: Talk und/oder Kieselerde und/oder Carbonaten und/oder komplexen Phosphaten, wie auf der Basis von $Na_2O$, $CaO$, $P_2O_5$ und $Al_2O_3$.

10. Verfahren nach irgendeinem der vorstehenden Ansprüche, dadurch gekennzeichnet, daß man auf Gra-

9

nulaten von Methionin und/oder Lysin eine Emulsion versprüht, bestehend aus einer Mischung von pH-sensiblem Polymer, Ethylcellulose. Stearinsäure, Natriumstearat und Wasser.

11. Verfahren nach Anspruch 3 und 10, dadurch gekennzeichnet, daß die Emulsion außerdem einen oder mehrere Zusatzstoffe enthält, ausgewählt unter den wie in Anspruch 9 definierten Füllstoffen, antistatischen Mitteln, plastifizierenden Mitteln, Farbstoffen und Mitteln für die Appetitlichkeit.

12. Verfahren nach irgendeinem der vorstehenden Ansprüche, dadurch gekennzeichnet, daß die Emulsion durch Mischen von 97 bis 60 Vol.-% einer wäßrigen Phase und 3 bis 40 Vol.-% einer organischen Phase hergestellt wird.

13. Verfahren nach Anspruch 12, dadurch gekennzeichnet, daß die wäßrige Phase 0 bis 10 Gew.-% eines Emulgators nach Anspruch 7 und/oder 0 bis 1 Gew.-% eines Alkalihydroxides oder Ammoniumhydroxid enthält, wobei die Summe der zwei mindestens 0,2 Gew.-% Natriumhydroxid-Äquivalent repräsentiert.

14. Verfahren nach Anspruch 3, dadurch gekennzeichnet, daß die organische Phase durch Dispergieren oder Auflösen einer festen Mischung erhalten wird, die folgende Bestandteile in Gewicht aufweist:
   - pH-sensibles Polymer 10 bis 70 %
   - eine oder mehrere hydrophobe Substanzen 30 bis 90 %
   - ein oder mehrere nicht wasserlösliche Polymere 0 bis 20 % in einem Lösungsmittel des oder der pH-sensiblen Polymere.

15. Verfahren nach Anspruch 3 und 14, dadurch gekennzeichnet, daß der Gewichts-Prozentsatz der verschiedenen Bestandteile zwischen den folgenden Grenzen liegt:
   - wäßrige Phase 50 bis 97 %
   - feste Mischung 2 bis 30 %
   - organisches Lösungsmittel 1 bis 20 %.

16. Verfahren nach Anspruch 15, dadurch gekennzeichnet, daß der Gewichts-Prozentsatz der verschiedenen Bestandteile zwischen den folgenden Grenzen liegt:
   - wäßrige Phase 72 bis 87 %
   - feste Mischung 10 bis 20 %
   - organisches Lösungsmittel 3 bis 8 %.